# EUROPÄISCHE PATENTSCHRIFT

(11) **EP 0 491 149 B1**
(45) Veröffentlichungstag und Bekanntmachung des Hinweises auf die Patenterteilung: **24.05.1995**
(21) Anmeldenummer: 91118687.2
(22) Anmeldetag: 02.11.1991
(51) Int. Cl.: A61B 17/16, B25D 17/08

(54) **Bohrfutter für eine insbesondere chirurgischen Zwecken dienende Bohrmaschine**
Drill chuck especially for surgical drill
Mandrin notamment pour perceuse chirurgicale

(30) Priorität: 19.12.1990 CH 4041/90
(43) Veröffentlichungstag der Anmeldung: 24.06.1992
(73) Patentinhaber: Synthes AG, Chur, CH-7002 Chur (CH)
(72) Erfinder: Zurbrügg, Andreas, CH-4056 Basel (CH); Schaufelberger, Jürg, CH-4147 Aesch (CH)
(74) Vertreter: Lusuardi, Werther Giovanni, Dr.

(56) Entgegenhaltungen:
- EP-A- 0 167 719
- EP-A- 0 339 910
- EP-A- 0 405 132
- WO-A-90/13421
- DE-A- 2 834 991
- US-A- 4 808 185

## Beschreibung

Die Erfindung betrifft ein Bohrfutter für insbesondere chirurgischen Zwecken dienende Bohrmaschine, gemäss dem Oberbegriff des Anspruchs 1.

Ein gattungsgemässes Bohrfutter ist aus der DE-A-28.34.992 BOR bekannt. Nachteilig bei diesem bekannten Bohrfutter ist einerseits die Tatsache, dass sich die Griffhülse mit dem Bohrer mitdreht, anderseits der Umstand, dass sämtliche Teile aus röntgenstrahlenundurchlässigem Material bestehen.

Weiter ist aus der EP-A 0 405 132 FRIGG nach veröffentlicht bereits ein Winkelaufsatz für eine Bohrmaschine bekannt, welcher teilweise aus röntgenstrahlendurchlässigem Material besteht. Dies ermöglicht dem Chirurgen eine ungehinderte Bildwandlerkontrolle, bei der ihm das Operationsfeld mittels der Röntgenbestrahlung entsprechend auf dem Bildschirm erscheint, ohne dass irgendwelche, durch die Bohrmaschine erzeugte Verdunkelungen auftreten würden. Damit eignet sich diese Bohrmaschine für verschiedene osteosynthetische Verfahren, so beispielsweise zum Anbringen von Verriegelungsbohrungen bei der Marknagelung. Insbesondere bei letzterer müssen wegen der anatomisch-geometrischen Verhältnisse solche Winkelgetriebe, die als sogenannte Winkelaufsätze bekannt sind, verwendet werden. Der Bohrer kann dabei fest oder aber vorteilhafterweise über eine geeignete Kupplung mit einer ausgangsseitigen Abtriebswelle des Winkelgetriebes verbunden sein. Bei einem operativen Eingriff kann es notwendig sein, den eingesetzten Bohrer raschmöglichst mit einem anderen, beispielsweise mit einem solchen grösseren Durchmessers, auszuwechseln. Ein solcher Wechsel ist bei dieser bekannten Bohrmaschine aber nicht optimal möglich.

Von diesem Stand der Technik ausgehend, liegt der Erfindung die Aufgabe zugrunde, ein Bohrfutter für eine insbesondere chirurgischen Zwecken dienende Bohrmaschine zu schaffen, welches eine einfache und rasche Handhabung vor allem beim Wechseln eines Bohrers erlaubt und dies bei vorausgesetzter einwandfreier permanenter Bildschirmüberwachung des durch Röntgenstrahlen vermittelten Bohrverlaufes.

Die Erfindung löst die gestellte Aufgabe mit einem Bohrfutter, welches die Merkmale des Anspruchs 1 aufweist. Damit sind dem Chirurgen nebst der durch die Röntgenstrahlendurchlässigkeit geschaffenen optimalen Bedingungen für eine Bildschirmüberwachung der Bohrarbeiten auch eine sehr einfache und schnelle Bohrmaschinen-Handhabung, primär beim Wechseln eines Bohrers, gewährleistet.

Das als Schnellwechselfutter ausgebildete Bohrfutter ist dabei vorzugsweise lösbar an einen Winkelaufsatz einer Bohrmaschine befestigt, um es leicht montieren, bzw. austauschen zu können. Überdies ist es vorteilhaft an diesen Winkelaufsatz befestigt, weil mit ihm für die oben genannten Bohrzwecke am idealsten gearbeitet werden kann. Im Prinzip könnte dieses Schnellwechselfutter aber auch an eine ohne Winkelaufsatz vorgesehene Bohrmaschine befestigt sein.

Weitere Einzelheiten und Vorteile des Bohrfutters nach der Erfindung sind anhand der Zeichnung in dem nachfolgenden Ausführungsbeispiel näher erläutert.
Es zeigt:
- Fig.1: einen Längsschnitt durch eine teilweise dargestellte Bohrmaschine mit einem erfindungsgemässen Bohrfutter, das teilweise in Ansicht gezeigt ist,
- Fig.2: einen Längsschnitt durch das Bohrfutter in einer den Bohrer freigebenden Position und
- Fig.3: einen Längsschnitt durch das Bohrfutter in der den Bohrer festhaltenden Position.

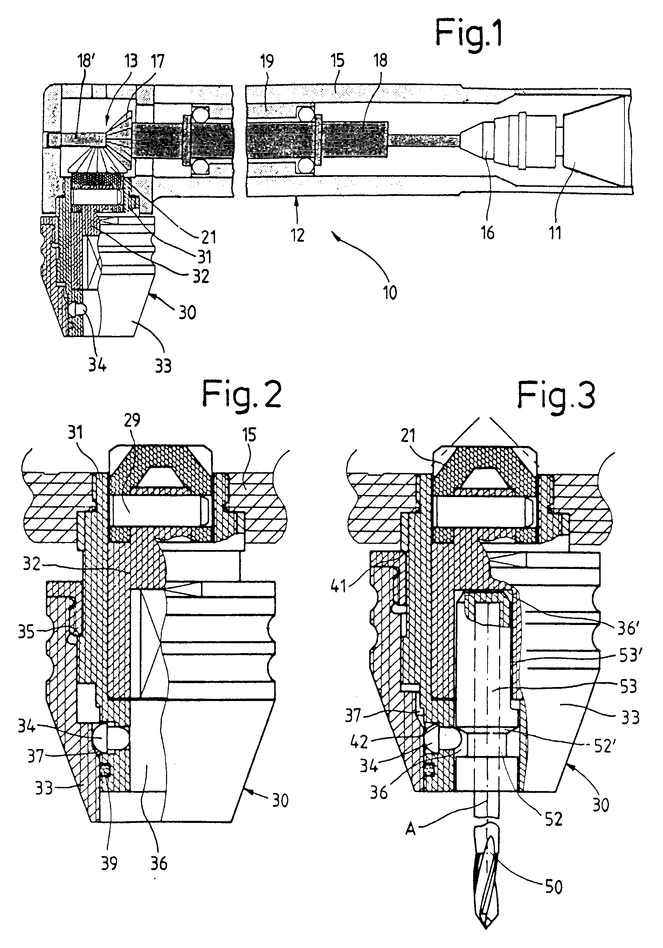

Die in Fig.1 gezeigte Bohrmaschine 10 besteht im wesentlichen aus einem teilweise dargestellten Antrieb 11, einem mit diesem drehverbundenen Winkelgetriebe 13 eines Winkelaufsatzes 12 sowie einem Schnellwechselfutter 30, das abtriebsseitig an das Winkelgetriebe 13 gekuppelt ist. Bei dem Antrieb 11 kann es sich bspw. um einen Elektromotor oder eine Pressluftturbine handeln und er weist dabei ein Spannfutter 16 auf, das mit einer Antriebswelle 18 des Winkelgetriebes 13 lösbar gekoppelt ist. Die Antriebswelle 13 ist mittels eines Lagers 19, das zum Beispiel aus zwei Wälzlagern besteht, drehbar in einem an den Antrieb 11 gekuppelten Gehäuse 15 des Winkelaufsatzes 12 gehalten. Zwecks Stabilisierung ist die Antriebswelle 18 an ihrem vorderen Ende 18' zusätzlich im Gehäuse 15 gelagert, und sie weist unmittelbar davor ein Kegelrad 17 auf, das zusammen mit einem mit ihm in Eingriff stehenden Kegelrad 21 das ebenfalls im Gehäuse 15 untergebrachte Winkelgetriebe 13 bildet.

Das abtriebsseitige Kegelrad 21 ist mit einer zur Antriebswelle 18 um 90° abgewinkelter Kupplungswelle 32 mittels eines Stiftes 29 drehverbunden. Diese Kupplungswelle 32 ist Bestandteil des erfindungsgemäss als Schnellwechselfutter 30 ausgebildeten Bohrfutters, das zudem aus einem am Gehäuse 15 festgeschraubten Futter 31, einer dieses umfassende und in Richtung der Bohrachse A hin- und herverschiebbar geführte Griffhülse 33 sowie aus drei um jeweils 120° zueinander versetzten, im Futter 31 quer zur Bohrachse A begrenzt verschiebbar angeordneten Verriegelungsstiften 34 besteht. Die Kupplungswelle 32 ist dabei im Futter 31 drehbar gelagert und diese bilden zusammen eine Oeffnung 36, in welcher ein Bohrer 50 einsteckbar ist. Das Schnellwechselfutter 30 ist dabei insgesamt aus Röntgenstrahlen durchlässigem Material gefertigt. Es haben sich dazu insbesondere Materialien wie Polyaetheraetherketon (PEEK), Polyamidimide (z.B. bekannt unter dem Namen TORLON) und/oder Polyoxymethylene (POM) als sehr geeignet erwiesen. Diese haben den Vorteil, als sie nicht nur röntgenstrahlendurchlässig, sondern auch selbstschmierend und bis 140° sterilisierbar sind. Fernerhin könnten auch Komposit-Kunststoffe, welche vorzugsweise faser-, gewebe- oder kugelverstärkt sind, oder spezielle keramische Werkstoffe eingesetzt werden.

Fig.2 und Fig.3 zeigen das Schnellwechselfutter 30 einerseits in der einen Bohrer 50 freigebenden und andererseits in der diesen festhaltenden Position. In ersterer Position ist die Griffhülse 33 auf dem Futter 31 an einen bohrerseitigen Anschlag 35 verschoben. Die im Futter 31 quer zur Bohrachse A verschiebbar angeordneten Verriegelungstifte 34 befinden sich dabei in zurückversetzter Position, d.h. sie ragen nicht in die den Bohrer 50 aufnehmende Oeffnung 36. Dies wird erreicht, indem eine in Richtung der Bohrachse A verlaufende Führungsbahn 37 innerhalb der Griffhülse 33 den jeweiliegen Verriegelungsstift 34 radial nach aussen begrenzt freigibt. Für das vorliegende Ausführungsbeispiel sind drei am Umfang des Futters 31 um je 120° versetzte Verriegelungsstifte 34 vorgesehen. Es könnten aber auch nur einer oder noch mehr als drei angeordnet sein.

In der den Bohrer 50 festhaltenden Position des Schnellwechselfutters 30 gemäss Fig.3 ist die Griffhülse 33 gegen einen antriebsseitigen Anschlag 41 verschoben. Die Verriegelungsstifte 34 ragen dabei in die Oeffnung 36 und damit in eine Ringnut 52 eines Bohrerschaftes 53, in dem der Bohrer 50 festgehalten ist. Durch das genannte Verschieben der Griffhülse 33 wird der jeweilige Verriegelungsstift 34 in der Führungsbahn 37 radial nach innen bis zu einem im Futter 31 vorgesehenen Anschlag 42 bewegt. Der halbkugelförmige Kopf des Verriegelungsstiftes 34 erlaubt eine einwandfreie Führung desselben in der Führungsbahn 37. Die Ringnut 52 des Bohrerschaftes 53 weist eine schräge Ringfläche 52' auf, die in Kontakt mit der abgerundeten Spitze des Verriegelungsstiftes 34 steht, wodurch der Bohrerschaft 53 stirnseitig an einer hinteren Stirnfläche 36' in der Oeffnung 36 anschlägt und damit der Bohrer 50 in seiner axialen Richtung fixiert ist. Der Bohrerschaft 53 hat überdies eine Anschlagfläche 53' und er ist damit formschlüssig in der Oeffnung 36 innerhalb der Kupplungswelle 32 gehalten und von dieser mitgenommen. Der Schaft 53 ist im Gegensatz zum Bohrer 50 aus röntgenstrahlendurchlässigem Material gefertigt und dadurch kann der genaue Bohrverlauf auf einem Bildschirm festgestellt und verfolgt werden, denn ausser dem Bohrer 50 lassen erfindungsgemäss die Bestandteile des Schnellwechselfutters 30 die Röntgenstrahlen praktisch verlustlos durch. Aus Verschleissgründen könnten höchstenfalls die Verriegelungsstifte 53 aus Stahl gefertigt sein.

Ferner ist noch zu erwähnen, dass die Griffhülse 33 aus einer eigentlichen Hülse sowie aus einem zweiteiligen, um das Futter 31 greifenden Ring besteht, wobei die Hülse auf den Ring aufgeschraubt und gesichert ist. Zwischen der Hülse und dem Futter ist an dem Bohrer zugekehrten Ende noch ein Abstreifring 39 vorgesehen, der eine Abdichtung insbesondere beim Verschieben der Griffhülse bewirkt.

Das beschriebene Futter und die in ihm gelagerte Kupplungswelle könnten im Prinzip auch aus einem Teil bestehen. Dieses müsste aber dann drehbar im Gehäuse 15 gelagert und entsprechend mit dem Kegelrad 21 rotativ verbunden sein. Folglich würde das ganze Bohrwechselfutter mitdrehen, währenddessen es in dem beschriebenen Ausführungsbeispiel stillsteht und nur gerade die Kupplungswelle 32 und der Bohrer 50 mit dem Schaft 53 drehen.

## Patentansprüche

1. Bohrfutter für insbesondere chirurgischen Zwecken dienende Bohrmaschine, das als Schnellwechselfutter (30) ausgebildet ist, wobei das Schnellwechselfutter (30) aus einem einen Bohrer (50) mitnehmenden ersten Teil (32), aus einer letzteren umfassenden und in Richtung der Bohrerachse (A) verschiebbaren Griffhülse (33) sowie aus mindestens einem quer zur Bohrerachse (A) begrenzt verschiebbar angeordneten Verriegelungselement (34) besteht, welches durch die Längsverschiebung der Griffhülse (33) einen im ersten Teil (32) mitgenommenen Bohrer (50) festhält oder freigibt, dadurch gekennzeichnet, dass das Schnellwechselfutter (30) insgesamt aus für Röntgenstrahlen durchlässigem Material besteht, dass die Griffhülse (33) zusätzlich einen den ersten Teil (32) umfassenden mit der Bohrmaschine feststellbaren zweiten Teil (31) umfasst und dass das mindestens eine Verriegelungselement (34) ein Verriegelungsstift ist, der im zweiten Teil (31) verschiebbar angeordnet ist.

2. Bohrfutter nach Anspruch 1, dadurch gekennzeichnet, dass das Schnellwechselfutter (30) vorzugsweise lösbar an einem Winkelaufsatz (12) einer Bohrmaschine (10) befestigbar ist.

3. Bohrfutter nach Anspruch 1, dadurch gekennzeichnet, dass das für Röntgenstrahlen durchlässige Material selbstschmierend und bis 140°C sterilisierbar ist.

4. Bohrfutter nach Anspruch 1, dadurch gekennzeichnet, dass die Griffhülse (33) auf dem zweiten Teil (31) von einem antriebsseitigen zu einem bohrerseitigen Anschlag (41,35) hin- und herverschiebbar geführt ist und innen eine in Richtung der Bohrerachse (A) verlaufende Führungsbahn (37) für den Verriegelungsstift (34) hat, durch welche sich dieser Verriegelungsstift (34) bei der bohrerseitigen Anschlagposition (35) der Griffhülse (33) in zurückversetzter Position befindet während er bei der antriebsseitigen Anschlagsposition (41) in eine Ringnut (52) eines Bohrerschaftes (53) des Bohrers (50) eingreift.

5. Bohrfutter nach Anspruch 4, dadurch gekennzeichnet, dass drei um je 120° versetzte Verriegelungsstifte (34) im zweiten Teil (31) vorgesehen sind, die jeweils einen in der Führungsbahn (37) laufenden halbkugelförmigen Kopf und eine abgerundete Spitze haben, wobei diese Spitze in der Ringnut (52) gegen eine schräge Ringfläche (52') und so den Bohrerschaft (53) gegen einen hinteren Anschlag (36' ) einer im ersten Teil, den Bohrer mitnehmenden Öffnung (36) drückt und damit den Bohrer (50) in seiner axialen Richtung fixiert ist.

6. Bohrfutter nach einem der Ansprüche 1 bis 5, dadurch gekennzeichnet, dass das für Röntgenstrahlen durchlässige Material aus verstärktem Polyetheretherketon (PEEK) und/oder Polyamidimid besteht.

7. Bohrfutter nach einem der Ansprüche 1 bis 5, dadurch gekennzeichnet, dass das für Röntgenstrahlen durchlässige Material aus verstärktem Polyoxymethylen (POM) besteht.

8. Bohrmaschine mit einem Bohrfutter nach Anspruch 1, dadurch gekennzeichnet, dass das zweite Teil (31) am Gehäuse (15) des Winkelaufsatzes (12) lösbar befestigt ist und eine drehbar gelagerte Kupplungswelle (32) aufweist, an deren einen Ende der Bohrer (50) in einer Öffnung (36) mitgenommen ist, während an deren anderem Ende ein Kegelrad (21) befestigt ist, das zusammen mit einem mit ihm im Eingriff stehenden Kegelrad (17), das auf einer um 90° versetzten Antriebswelle (18) sitzt, das Winkelgetriebe (13) des Winkelaufsatzes (12) bildet.

9. Bohrer mit einem Bohrfutter nach einem der Ansprüche 1 - 7, dadurch gekennzeichnet, dass der aus röntgenstrahlenundurchlässigem Material gefertigte Bohrer (50) in einem separaten Bohrerschaft (53) gehalten ist, letzterer hingegen aus röntgenstrahlendurchlässigem Material besteht und form- und/oder kraftschlüssig in einer Öffnung (36) des ersten Teils (32) gehalten ist.

## Claims

1. Drill chuck for a drilling machine intended particularly for surgical purposes, which is designed as a quick-change chuck (30), whereby the quick-change chuck (30) consists of a first part (32) catching a drill (50), a gripper sleeve (33) encompassing said first part (32) and movable in direction of the drill axis (A), and at least one locking pin (34) partially transversely movable to said drill axis (A), said drill chuck (30) holding or releasing a drill (50) drawn into the first part (32) by means of the longitudinal movement of the gripper sleeve (33), characterized in that the quick-change chuck (30) is made entirely of a material that is permeable by x-rays, that the gripper sleeve (33) comprises additionally a second part (31) which encompasses said first part (32) and which is fixable to the drilling machine, and that said at least one locking pin (34) is a locking pin movably arranged in said second part (31).

2. Drill chuck according to claim 1, characterized in that the quick-change chuck (30) is attached preferably in removable fashion to an angle jaw (12) of a drilling machine (10).

3. Drill chuck according to claim 1, characterized in that the material permeable by x-rays is self-lubricating and can be sterilized up to 140 °C.

4. Drill chuck according to claim 1, characterized in that the gripper sleeve (33) is guided on the second part (31) from a drive-side to a drill-side stop (41,35) by being slid back and forth, and has inside it a guide track (37), running in the direction of the drill axis (A), for the locking pin (34), by means of which this locking pin (34) is in drawn-back position when the gripper sleeve (33) is in drill-side stop position (35), whereas in drive-side stop position (41) it engages in an annular groove (52) of a drill shaft (53) of the drill (50).

5. Drill chuck according to claim 4, characterized in that there are in the second part (31) three locking pins (34), offset at 120° each, each of which locking pins (34) has a rounded tip and a semi-spherical head that runs in the guide track (37); the rounded tip in the annular groove (52) presses against an oblique groove surface (52') and thus presses the drill shaft (53) against a rear stop (36') of an opening (36) catching the drill in the first part (32), whereby the drill (50) is fixed in its axial direction.

6. Drill chuck according to one of claims 1 to 5, characterized in that the material permeable by x-rays consists of reinforced polyetheretherketon (PEEK) and/or polyamidimid.

7. Drill chuck according to one of claims 1 to 5, characterized in that the material permeable by x-rays consists of reinforced polyoxymethylene (POM).

8. Drilling machine with a drill chuck according to claim 1, characterized in that the second part (31) is removably attached to the housing (15) of the angle jaw (12), and that the second part (31) has a rotationally housed coupling shaft (32), at one end of which the drill (50) is drawn into an opening (36), whereas a bevel gear (21) is attached at its other end, which said bevel gear (21), together with a bevel gear (17) that engages with it and which is positioned on a drive shaft (18) offset at a 90° angle, forms the angle gear (13) of the angle jaw (12).

9. Drill with a drill chuck according to one of the claims 1 - 7, characterized in that the drill (50), which is made of a material that is not permeable by x-rays, is held in the separate drill shaft (53); the drill shaft (53) in contrast is made of a material that is permeable by x-rays, and is held solidly or by friction in a opening (36) of the first part (32).

## Revendications

1. Mandrin, en particulier pour foreuse chirurgicale, réalisé comme mandrin à échange rapide (30), le mandrin à échange rapide (30) étant constitué d'une première partie (32) entraînant un foret (50), d'un manchon d'accrochage (33) entourant ladite première partie et pouvant coulisser dans la direction de l'axe de forage (A), ainsi que d'au moins un élément de verrouillage (34) disposé de manière à pouvoir coulisser de manière limitée transversalement à l'axe du foret (A), et qui maintient ou libère par le coulissement latéral du manchon d'accrochage (33) un foret (50) entraîné dans la première partie (32), caractérisé en ce que le mandrin à échange rapide (30) est globalement constitué de matériau transparent aux rayons X, en ce que le manchon d'accrochage (33) comprend en plus une seconde partie (31) entourant la première partie (32) et pouvant être fixé à la foreuse, et en ce que l'élément de verrouillage (34) au moins présent est une tige de verrouillage qui est installée à coulissement dans la seconde partie (31).

2. Mandrin selon la revendication 1, caractérisé en ce que le mandrin à échange rapide (30) peut être fixé, de préférence de manière libérable, sur un renvoi d'angle (12) d'une foreuse (10).

3. Mandrin selon la revendication 1, caractérisé en ce que le matériau transparent aux rayons X est auto-lubrifiant et peut être stérilisé jusqu'à 140°C.

4. Mandrin selon la revendication 1, caractérisé en ce que le manchon d'accrochage (33) est guidé de manière à coulisser en va-et-vient sur la seconde partie (31), depuis une butée (41) située côté entraînement jusqu'à une butée (35) située côté foret, et présente à l'intérieur une piste de guidage (37) de la tige de verrouillage (34), s'étendant dans la direction de l'axe du foret (A), grâce à laquelle cette tige de verrouillage (34) se trouve dans la position reculée lorsque le manchon d'accrochage (33) se trouve dans la position de butée (35) située côté foret, alors que dans la position de butée (41) située côté entraînement, ladite tige de verrouillage pénètre dans une rainure annulaire (52) d'une tige de foret (53) du foret (50).

5. Mandrin selon la revendication 4, caractérisé en ce que dans la seconde partie (31) sont prévues trois tiges de verrouillage (34) décalées chacune de 120°, qui présentent chacune une tête hémisphérique se déplaçant dans la piste de guidage (37) et un sommet arrondi, ce sommet arrondi exerçant une poussée dans la rainure annulaire (52) contre une surface annulaire oblique (52'), et repoussant ainsi la tige de foret (53) contre une butée arrière (36') d'une ouverture (36) de la première partie, reprenant le foret, et qu'ainsi le foret (50) est immobilisé dans sa direction axiale.

6. Mandrin selon l'une quelconque des revendications 1 à 5, caractérisé en ce que le matériau transparent aux rayons X est constitué de polyétheréthercétone (PEEC) et/ou de polyamideimide renforcés.

7. Mandrin selon l'une quelconque des revendications 1 à 5, caractérisé en ce que le matériau transparent aux rayons X est constitué de polyoxyméthylène (POM) renforcé.

8. Foreuse comportant un mandrin selon la revendication 1, caractérisée en ce que la seconde partie (31) est fixée de manière libérable au boitier (15) du renvoi d'angle (12), et présente un arbre d'accouplement (32) monté à rotation, à une des extrémités duquel le foret (50) est repris dans une ouverture (36), tandis qu'à son autre extrémité est fixée une roue conique (21), qui forme le mécanisme de renvoi d'angle (13) du renvoi d'angle (12) avec une roue conique (17) s'engrenant sur ladite roue conique (21) et placée sur un arbre d'entraînement (18) décalé de 90°.

9. Foret avec un mandrin selon l'une quelconque des revendications 1 à 7, caractérisé en ce que le foret (50) fabriqué en un matériau opaque aux rayons X est maintenu dans une tige de foret (53) séparée, mais que ladite tige de foret (53) est constituée d'un matériau transparent aux rayons X et est maintenue par correspondance de forme et/ou par action mécanique dans une ouverture (36) de la première partie (32).
